# EUROPEAN PATENT APPLICATION

(11) **EP 4 548 898 A1**
(43) Date of publication of application: **07.05.2025**
(21) Application number: 23831583.2
(22) Date of filing: 29.06.2023
(51) Int. Cl.: A61H 23/02, A61B 10/00, A61B 5/00, A61B 5/026

(54) **BODY SURFACE ULCER HEALING PROMOTING DEVICE**

(30) Priority: 01.07.2022 JP 2022107292
(71) Applicant: The University of Tokyo, Bunkyo-ku, Tokyo 113-8654 (JP)
(72) Inventor: NAKAGAMI, Gojiro, Tokyo 113-8654 (JP); HABA, Daijiro, Tokyo 113-8654 (JP); SEKINO, Masaki, Tokyo 113-8654 (JP); MINAMI, Tsuyoshi, Tokyo 113-8654 (JP); SANADA, Hiromi, Tokyo 113-8654 (JP); MINEMATSU, Takeo, Tokyo 113-8654 (JP)
(74) Representative: Mathys & Squire
(86) International application number: PCT/JP2023/024188
(87) International publication number: WO 2024/005144

(57) **Abstract**

A body surface ulcer healing promotion device is provided, which includes: a glucose concentration sensor 11 that detects a glucose concentration in an exudate from an ulcer 2 on a body surface 1; vibrators 21A, 21B, 21C that apply vibration to the ulcer 2; and a control device 31 that controls the vibration of the vibrators 21A, 21B, 21C on a basis of the glucose concentration in the exudate.

## Description

### Technical Field

The present invention relates to a treatment technique, and to a body surface ulcer healing promotion device.

### Background Art

Diabetes and complications therefrom are main nontraumatic causes of lower limb amputation. In a large number of cases, foot ulcers develop before surgery for lower limb amputation. Correction of the hyperglycemia is generally quite difficult at a stage where a foot ulcer has developed. It is thought that only two-thirds of foot ulcers ultimately heal, with the remainder resulting in some form of surgical amputation. Foot ulcers and surgical amputation have a significant impact on the lives of patients (refer, for example, to Patent Literature 1 to Patent Literature 3). Methods for treating foot ulcers include the local administration of platelet-derived growth factor.

### Citation List

### Patent Literature

Patent Literature 1: Japanese Patent No. 5,715,326
Patent Literature 2: Japanese Patent No. 5,252,585
Patent Literature 3: Japanese Patent No. 5,558,573

### Summary of Invention

### Technical Problem

There are few options with regard to methods for treating ulcers on the surface of a body, and there is desire for a means to promote the healing of ulcers while allowing patients to live normal daily lives. An object of the present invention is therefore to provide a device that promotes the healing of ulcers on the surface of a body.

### Solution to Problem

The body surface ulcer healing promotion device according to an aspect of the present invention contains a glucose concentration sensor that detects the glucose concentration in an exudate from an ulcer on the body surface, a vibrator for applying vibration to the ulcer, and a control device that controls the vibration of the vibrator on a basis of the glucose concentration in the exudate.

The ulcer for the body surface ulcer healing promotion device may be an ulcer caused by diabetes.

The ulcer for the body surface ulcer healing promotion device may be a foot ulcer caused by diabetes.

The body surface ulcer healing promotion device may be provided with a plurality of vibrators.

The control device in the body surface ulcer healing promotion device may control the vibration of a vibrator such that cellular arginine production is promoted.

The control device in the body surface ulcer healing promotion device may control the vibration of a vibrator such that the glucose concentration in the exudate becomes equal to or greater than a concentration at which blood flow is increased.

The control device in the body surface ulcer healing promotion device may cause a vibrator to vibrate until the glucose concentration in the exudate becomes equal to or less than a concentration at which the amount of cellular arginine production becomes equal to or greater than a threshold value.

The body surface ulcer healing promotion device may further include a blood flow sensor that detects blood flow in the vicinity of the ulcer, and the control device may control the vibration of a vibrator on a basis of the blood flow.

The control device in the body surface ulcer healing promotion device may control the vibration of a vibrator so as to increase the blood flow.

### Advantageous Effect of Invention

The present invention can provide a device that promotes the healing of ulcers on the surface of the body.

### Brief Description of Drawings

[Figure 1] Figure 1 is a schematic diagram that shows a body surface ulcer healing promotion device according to an embodiment.
[Figure 2] Figure 2 is a schematic diagram that shows the relationship between hyperglycemia and metabolism according to an embodiment.
[Figure 3] Figure 3 is a schematic diagram that shows the relationship between hyperglycemia and metabolism according to an embodiment.
[Figure 4] Figure 4 is a graph that shows the amount of uptake of 2-deoxyglucose according to an example.
[Figure 5] Figure 5 is a graph that shows the amount of uptake of 2-deoxyglucose according to an example.
[Figure 6] Figure 6 is a graph that shows the amount of uptake of 2-deoxyglucose according to an example.
[Figure 7] Figure 7 is a graph that shows the amount of uptake of 2-deoxyglucose according to an example.
[Figure 8] Figure 8 is a schematic diagram that shows GLUT4 translocation according to an example.
[Figure 9] Figure 9 contains a schematic diagram and photographs that show GLUT4 translocation according to an example.
[Figure 10] Figure 10 contains photographs that indicate the presence/absence of GLUT4 translocation according to an example.
[Figure 11] Figure 11 is a graph of the ratio of the intensity of GLUT4-derived fluorescence in the cell membrane to the intensity of GLUT4-derived fluorescence in the cytoplasm, according to an example.
[Figure 12] Figure 12 contains photographs of a rat, according to an example.
[Figure 13] Figure 13 contains photographs of wound sites on rats, according to an example.
[Figure 14] Figure 14 contains photographs of wound sites on rats, according to an example.
[Figure 15] Figure 15 is a graph of the wound site area in rats, according to an example.
[Figure 16] Figure 16 contains photographs of wound site tissue in rats, according to an example.
[Figure 17] Figure 17 contains photographs of wound site tissue in rats, according to an example.
[Figure 18] Figure 18 is a graph of the blood volume in rats, according to an example.
[Figure 19] Figure 19 is a circuit diagram of a device for applying vibration according to an example.
[Figure 20] Figure 20 contains photographs of wound sites in rats, according to an example.
[Figure 21] Figure 21 is a graph of the wound site area in rats, according to an example.
[Figure 22] Figure 22 is a graph of the blood volume in rats, according to an example.
[Figure 23] Figure 23 contains photographs of the tissue at the wound site in rats, according to an example.
[Figure 24] Figure 24 is a graph of gene expression levels in rats, according to an example.
[Figure 25] Figure 25 is a graph of gene expression levels in rats, according to an example.
[Figure 26] Figure 26 is a graph of gene expression levels in rats, according to an example.
[Figure 27] Figure 27 contains photographs of rat wound site tissue stained using anti-PTX3 antibody, according to an example.
[Figure 28] Figure 28 is a graph of the value provided by dividing the number of CD68 positive cells by the number of CD163 positive cells (M1/M2 macrophage ratio), according to an example.

### Description of Embodiments

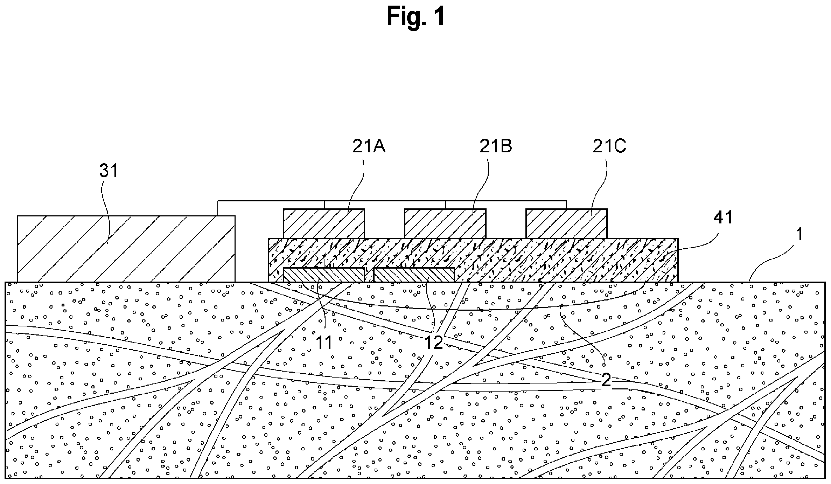

As shown in Figure 1, the body surface ulcer healing promotion device according to an embodiment has a glucose concentration sensor 11 that detects the glucose concentration in an exudate from an ulcer 2 on a body surface 1; vibrators 21A, 21B, 21C that apply vibration to the ulcer 2; and a control device 31 that controls the vibration of the vibrators 21A, 21B, 21C based on the glucose concentration in the exudate.

The following may be used for the vibrators 21A, 21B, 21C: a motor, a pneumatic device, a transducer material, a piezoelectric foil, a voice coil, or a piezoelectric actuator. The number of vibrators 21A, 21B, 21C provided in the body surface ulcer healing promotion device according to the embodiment is not particularly limited, and may be one or may be a plurality. The vibrators 21A, 21B, 21C are arranged, for example, so as to surround the ulcer 2 on the patient. The vibrators 21A, 21B, 21C generate, for example, low-frequency vibrations.

The low frequency is not particularly limited, but may be, for example, at least 20 Hz and not more than 100 Hz, at least 30 Hz and not more than 90 Hz, at least 40 Hz and not more than 80 Hz, at least 40 Hz and not more than 70 Hz, or at least 40 Hz and not more than 60 Hz. The vibration intensity is not particularly limited, but may be, for example, at least 100 mVpp and not more than 3000 mVpp, at least 500 mVpp and not more than 2500 mVpp, at least 500 mVpp and not more than 2000 mVpp, or at least 800 mVpp and not more than 2000 mVpp.

The vibrators 21A, 21B, 21C may be placed on the body surface 1 via an interposed dressing 41. The material of the dressing 41 can be exemplified by silicones, polyurethanes, hydrophilic membranes, hydrophilic fibers, hydrocolloids, hydrogels, cellulose acetate, and polyvinyl alcohol.

The glucose concentration sensor 11 is placed, for example, in contact with an ulcer 2 on the patient. The principle by which the glucose concentration sensor 11 detects the glucose concentration is not particularly limited. For example, the glucose concentration sensor 11 may have a membrane on which glucose oxidase is immobilized, a platinum electrode, and a silver electrode. When glucose is degraded into gluconic acid and hydrogen peroxide by the glucose oxidase, the hydrogen peroxide is then oxidized at the platinum electrode and reduced at the silver electrode. The current thereby generated at the electrodes is proportional to the glucose concentration. The glucose concentration sensor 11 detects the glucose concentration in the exudate from the ulcer 2 and transmits the glucose concentration in the exudate of the ulcer 2 to the control device 31.

The control device 31 controls the vibration of the vibrators 21A, 21B, 21C such that the vibration of the vibrators 21A, 21B, 21C satisfies a condition that can promote cellular arginine production. The intensity of the vibrations and the frequency of the vibrations are examples of conditions that can promote cellular arginine production.

The control device 31 may store a preliminarily acquired relationship between the glucose concentration in the exudate and the amount of intracellular arginine production. The control device 31 may also store a preliminarily acquired threshold value for the amount of intracellular arginine production that is required for the appropriate synthesis of nitric oxide. In general, the glucose concentration in the exudate and the amount of intracellular arginine production reside in a negative proportional relationship. Thus, when the glucose concentration in the exudate is high, the amount of intracellular arginine production is low, and when the glucose concentration in the exudate is low, the amount of intracellular arginine production is high.

The control device 31 controls the vibration of the vibrators 21A, 21B, 21C such that the glucose concentration in the exudate, as detected by the glucose concentration sensor 11, is equal to or less than a concentration at which the amount of cellular arginine production becomes equal to or greater than the threshold value. The control device 31 may induce the vibration of the vibrators 21A, 21B, 21C until the glucose concentration in the exudate, as detected by the glucose concentration sensor 11, becomes equal to or less than a concentration at which the amount of cellular arginine production becomes equal to or greater than the threshold value.

The control device 31 may store a preliminarily acquired relationship between the glucose concentration in the exudate and blood flow. The control device 31 may also store a preliminarily acquired threshold value for a suitable blood flow. In general, the glucose concentration in the exudate and the blood flow reside in a negative proportional relationship. Thus, the blood flow is small when the glucose concentration in the exudate is high, and the blood flow is large when the glucose concentration in the exudate is low.

The control device 31 controls the vibrators 21A, 21B, 21C such that the glucose concentration in the exudate, as detected by the glucose concentration sensor 11, becomes equal to or less than the concentration at which the blood flow becomes equal to or greater than the threshold value. The control device 31 may induce the vibration of the vibrators 21A, 21B, 21C until the glucose concentration in the exudate, as detected by the glucose concentration sensor 11, becomes equal to or less than the concentration at which the blood flow becomes equal to or greater than the threshold value.

The body surface ulcer healing promotion device according to an embodiment may additionally have a blood flow sensor 12 that detects blood flow in the vicinity of the ulcer. The blood flow detection principle used by the blood flow sensor 12 is not particularly limited. For example, the blood flow sensor 12 may have a light source and a light receiving element. Laser light emitted from the light source toward the human body is then scattered by the red blood cells in the blood vessels. The blood flow sensor 12 receives the scattered light with the light receiving element and detects the blood flow from the frequency spectrum of the scattered light. The blood flow sensor 12 transmits the detected blood flow to the control device 31.

The control device 31 may control the vibration of the vibrators 21A, 21B, 21C based on the blood flow. For example, the control device 31 may control the vibration of the vibrators 21A, 21B, 21C such that a condition that promotes an increase in blood flow is satisfied. The intensity of the vibrations and the frequency of the vibrations are examples of conditions that can promote an increase in blood flow.

The ulcer may be an ulcer caused by diabetes. The ulcer may be a foot ulcer. In the human body, glucose taken into cells from blood vessels is normally used to produce ATP in the glycolysis system and the citric acid cycle. However, when the glucose uptake into cells is increased due to hyperglycemia, the glucose is taken into the polyol metabolic pathway.

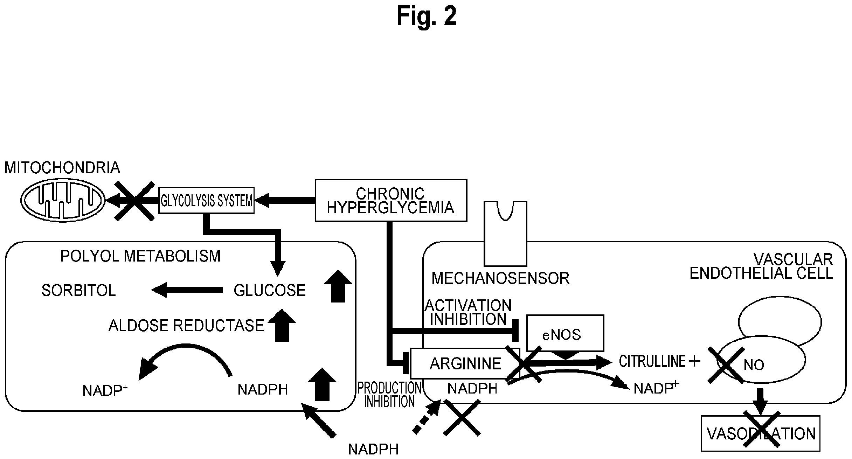

As shown in Figure 2, glucose is converted by aldose reductase in the polyol metabolic pathway to sorbitol. When the aldose reductase activity in the polyol metabolic pathway is increased, the coenzyme NADPH (reduced nicotinamide adenine dinucleotide phosphate) is consumed. NADPH is also used in vascular endothelial cells in the synthesis of nitric oxide (NO) using arginine, but nitric oxide synthesis declines when NADPH is consumed in the polyol metabolic pathway. In addition, arginine production is inhibited when the glucose concentration is high. Nitric oxide synthesis decreases when arginine production is inhibited. The decrease in nitric oxide brings about a decrease in blood flow and ischemia.

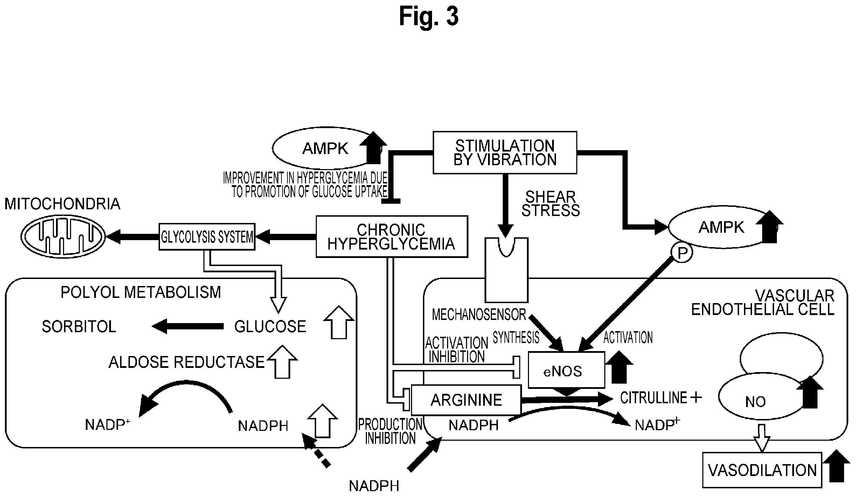

Without being bound by theory, it is thought that, through the application of vibrations to an ulcer 2 by the body surface ulcer healing promotion device according to the embodiments, the AMPK (AMP-activated protein kinase) is, as shown in Figure 3, activated in the adipocytes in the vicinity of the ulcer 2 and the insulin resistance of the adipocytes is improved. It is also thought that the improvement in the insulin resistance of the adipocytes results in an optimization of the glucose concentration in the vicinity of the adipocytes, suitable synthesis of nitric oxide in vascular endothelial cells, and a promotion of blood flow. Diabetic foot ulcers readily form in locations with little muscle, such as the metatarsal bones and heel. In addition, diabetic patients also present muscle atrophy due to peripheral neuropathy and have poor muscle function. Due to this, it is thought that the body surface ulcer healing promotion device according to the embodiments activates AMPK in adipocytes in the vicinity of the ulcer 2.

Furthermore, without being bound by theory, it is thought that the mechanosensors in vascular endothelial cells respond to the mechanical stress generated by the body surface ulcer healing promotion device according to the embodiments and promote the synthesis of nitric oxide synthase (NOS) in the vascular endothelial cells. It is also thought that the activation of AMPK in the adipocytes brings about the activation of nitric oxide synthase in the vascular endothelial cells. It is thought that the promotion of synthesis of nitric oxide synthase and its activation leads to a suitable synthesis of nitric oxide in the vascular endothelial cells, thus promoting blood flow.

The body surface ulcer healing promotion device according to the embodiments can be miniaturized, and due to this can be continuously fixed to the body surface of the patient while the patient can live an independent daily life. In addition, the insulin resistance of a patient is not constant and can fluctuate. The body surface ulcer healing promotion device according to the embodiments controls the vibration of the vibrators 21A, 21B, 21C while detecting the glucose concentration in the exudate, which fluctuates depending on the insulin resistance, and due to this the imposition of an unnecessary burden on the patient can be avoided.

The stages of ulcer healing are divided into the inflammatory phase and the proliferation phase. In the inflammatory phase, neutrophils and M1 inflammatory macrophages infiltrate into the ulcer and edema is produced. In the proliferation phase, fibroblasts migrate from the periphery of the ulcer to reconstruct the extracellular matrix, angiogenesis occurs, and granulation tissue is formed. In chronic hyperglycemic diabetic patients, the persistence of inflammation and the inability to transition to the proliferation phase is the cause of refractory foot ulcers. The body surface ulcer healing promotion device according to the embodiments, by locally vibrating the vicinity of the ulcer and promoting early wound contraction, causes an early transition of the ulcer to the proliferation phase without prolonging inflammation.

### (Example 1)

3T3-L1 adipocytes were prepared by differentiation into adipocytes and then allowing 8 days to elapse. The adipocytes were divided into a group that received vibration and a group that did not receive vibration, and each were cultured. The vibration group received a 50 Hz vibration for 40 minutes each day, at an intensity of 600 mVpp, 800 mVpp, 1000 mVpp, 1500 mVpp, or 2000 mVpp.

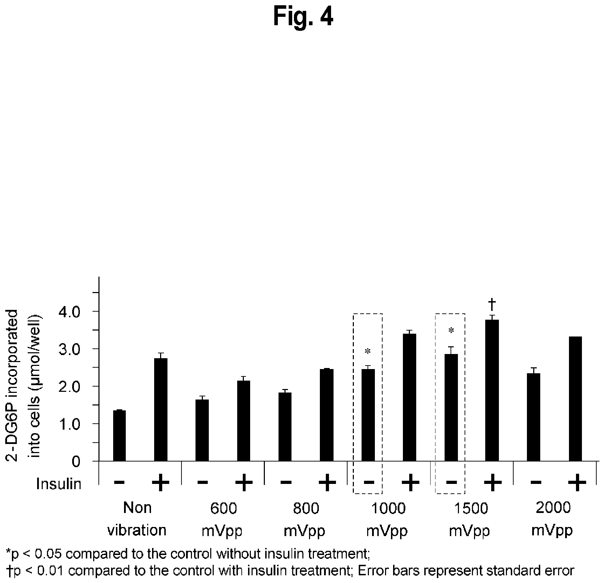

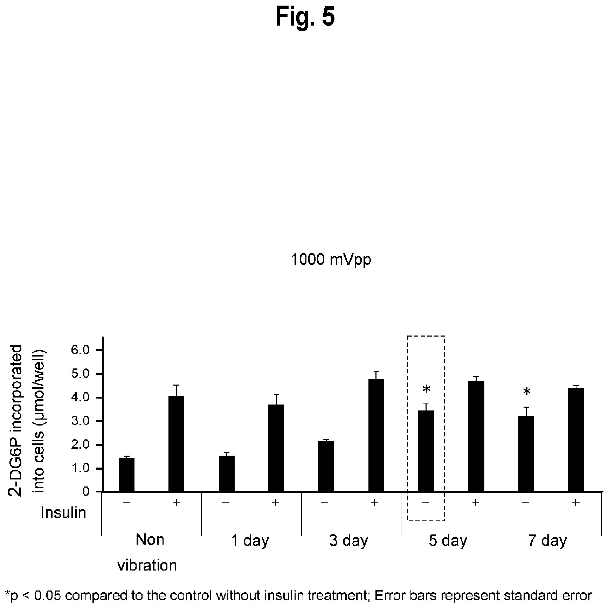

After the elapse of 7 days, and as shown in Figure 4, the vibration group had a higher amount of 2-deoxyglucose uptake by the adipocytes than did the nonvibration group. In addition, the uptake of 2-deoxyglucose by the adipocytes was examined for 7 days. As a result, as shown in Figure 5, the amount of 2-deoxyglucose uptake by the adipocytes increased over time when vibration was applied.

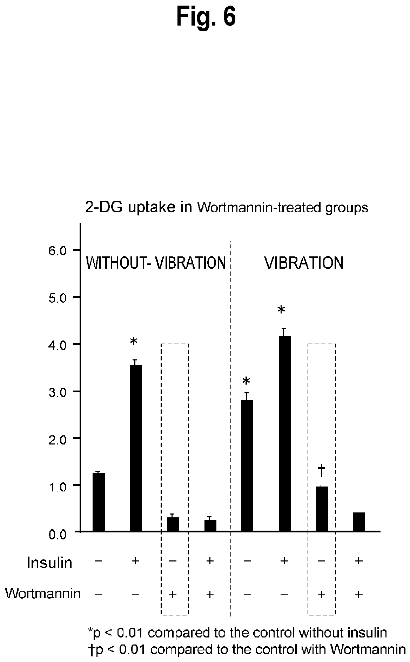

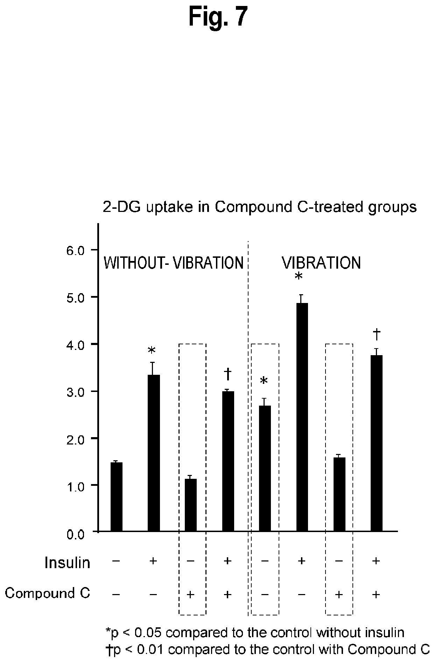

When wortmannin, an insulin-mediated glucose uptake pathway inhibitor, was administered to the adipocytes, as shown in Figure 6, the wortmannin did not significantly inhibit the vibration-induced effect of promoting 2-deoxyglucose uptake. On the other hand, when the AMPK inhibitor Compound C was administered to the adipocytes, as shown in Figure 7, Compound C significantly inhibited the vibration-induced effect of promoting 2-deoxyglucose uptake. This result indicates that the vibration-induced effect of promoting 2-deoxyglucose uptake is due to the activation of AMPK by the vibration.

### (Example 2)

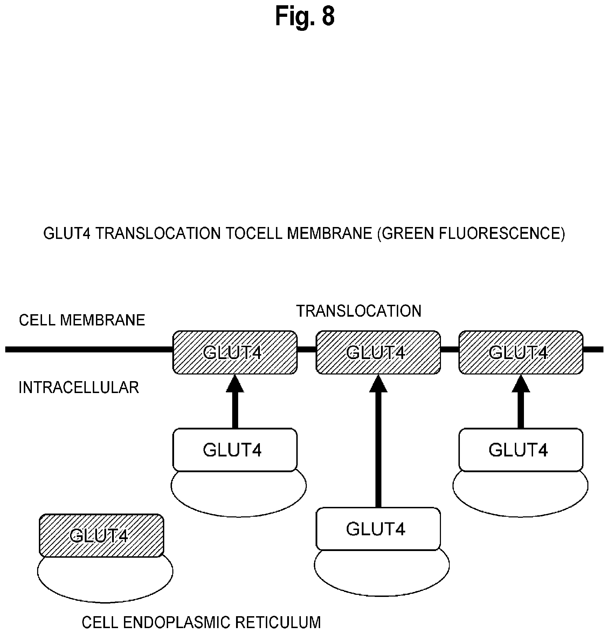

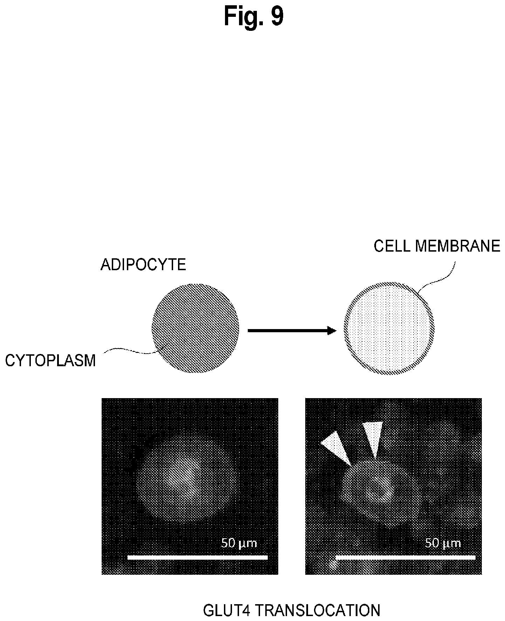

The cellular uptake of blood glucose, which is promoted by insulin and exercise, is mediated by the protein GLUT4, a glucose transporter. As shown in Figure 8 and Figure 9, GLUT4 is ordinarily stored in GLUT4 storage compartments in the cytoplasm. When stimulation from insulin or exercise is applied to the cell, GLUT4 is transported from the storage compartments to the cell membrane. It is known that this phenomenon, referred to as GLUT4 translocation, is inhibited by the insulin resistance caused by type 2 diabetes. GLUT4 translocation can be observed by the immunofluorescence staining of GLUT4.

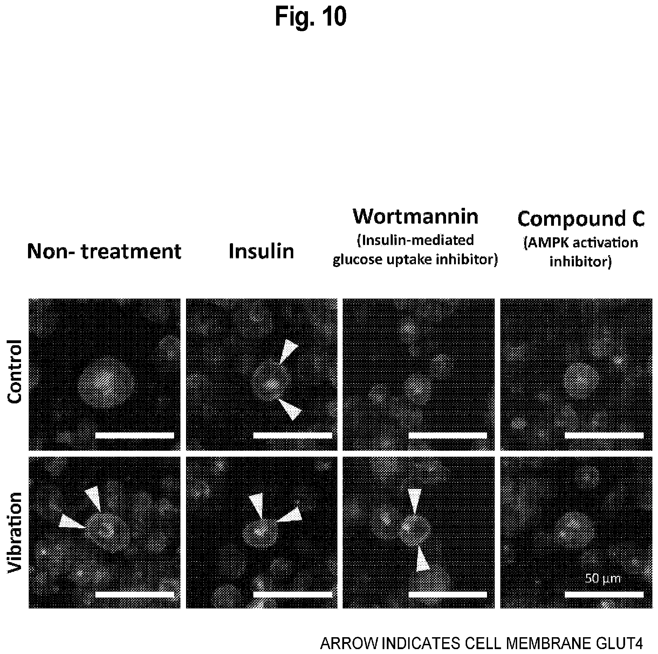

Observations were carried out as to whether GLUT4 translocation was produced in 3T3-L1 adipocytes by vibration for 5 days at 50 Hz, 1000 to 1500 mVpp, 40 minutes/day. As shown in Figure 10, in cells that were not given insulin or inhibitors, GLUT4 was present in the cytoplasm in cells that were not subjected to vibration, while GLUT4 was skewed to the cell membrane in cells that received vibration. In cells that received insulin but did not receive an inhibitor, GLUT4 was skewed to the cell membrane both in the cells that did not receive vibration and in the cells that received vibration. In cells that were given wortmannin, GLUT4 was present in the cytoplasm in the cells did not receive vibration, but was skewed to the cell membrane in cells that received vibration. In cells that received Compound C, GLUT4 was present in the cytoplasm in the cells that did not receive vibration and GLUT4 was also present in the cytoplasm in the cells that received vibration.

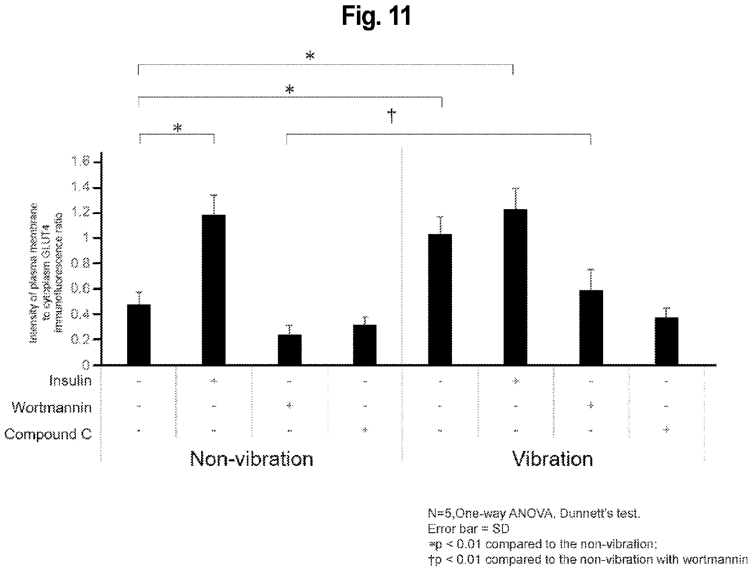

Figure 11 provides a graph of the ratio of the fluorescence intensity in the cell membrane to the fluorescence intensity in the cytoplasm. In cells that received Compound C, no significant difference was observed between the cells that received vibration and the cells that did not receive vibration. In cells that received wortmannin, a significant difference was observed between the cells that did not receive vibration and the cells that received vibration. This result also indicates that the vibration-induced effect of promoting 2-deoxyglucose uptake is due to the activation of AMPK by the vibration.

### (Example 3)

Seven-week-old male SD rats were prepared. 55 mg/kg streptozotocin was administered intraperitoneally to the rats, and rats with blood glucose values of at least 300 mg/dL after 7 and 14 days were selected as diabetic model rats.

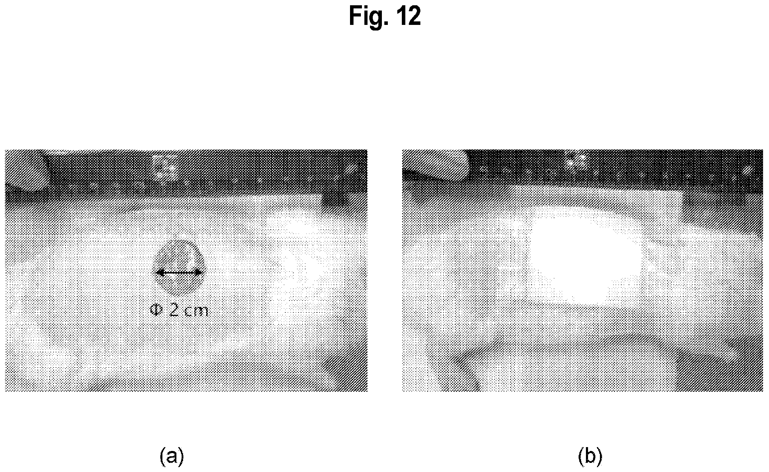

As shown in Figure 12, a full-thickness excisional wound with a diameter of 2 cm was formed on the flank of the anesthetized diabetic model rat; a dressing (Foamlite, ConvaTec) was applied to the wound site; and the torso was fixed with gauze. The dressing was changed daily.

This was followed by the daily local application, while the diabetic model rat was asleep under isoflurane inhalation anesthesia, of a low-frequency vibration for 40 minutes to the wound site for 14 days; the vibrator was placed on the gauze over the wound site. The frequency of the vibration was 50 Hz, and the vibration intensity was 0 mVpp, 300 mVpp, 600 mVpp, or 1000 mVpp.

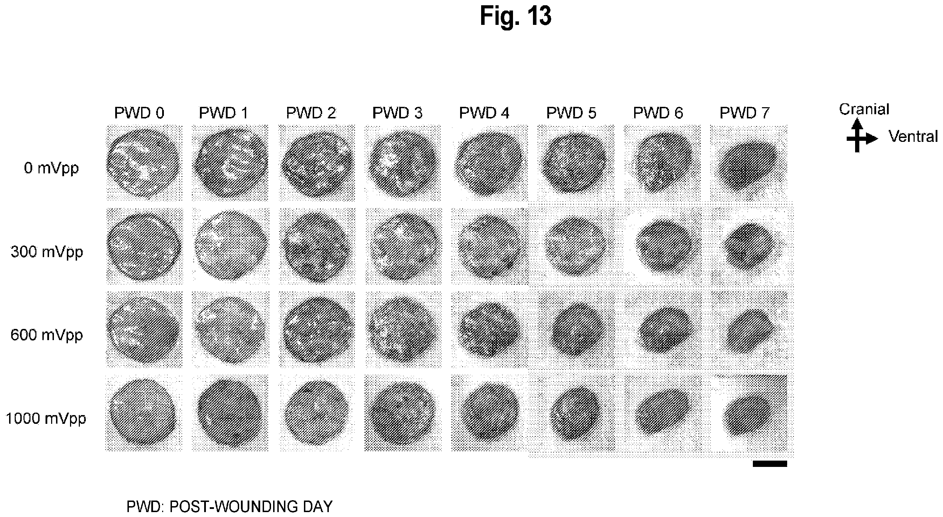

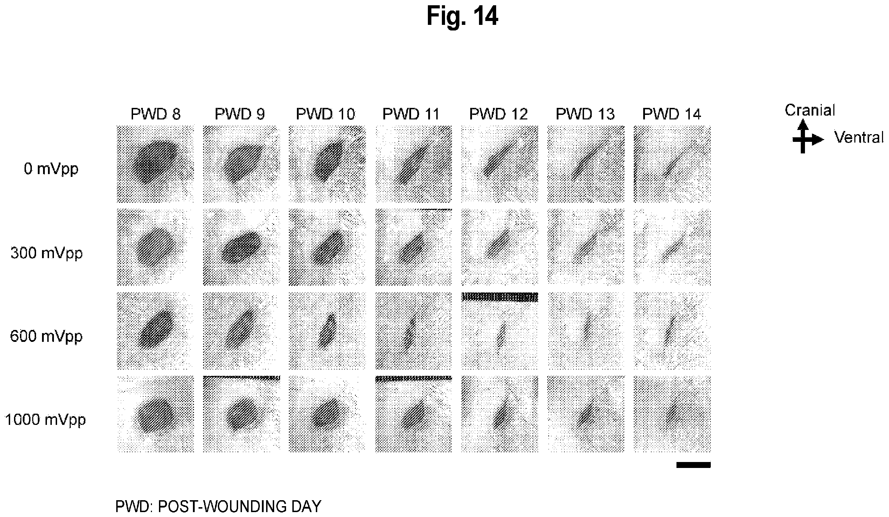

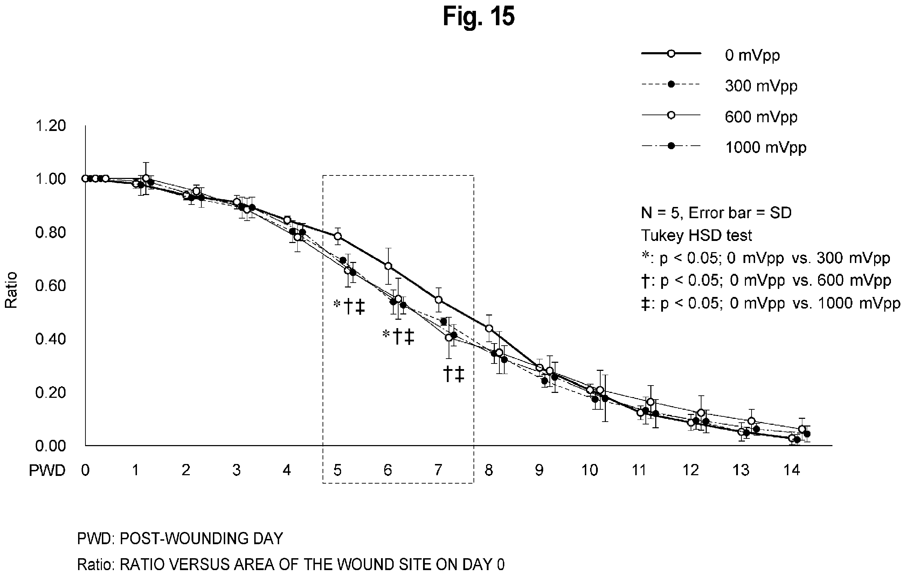

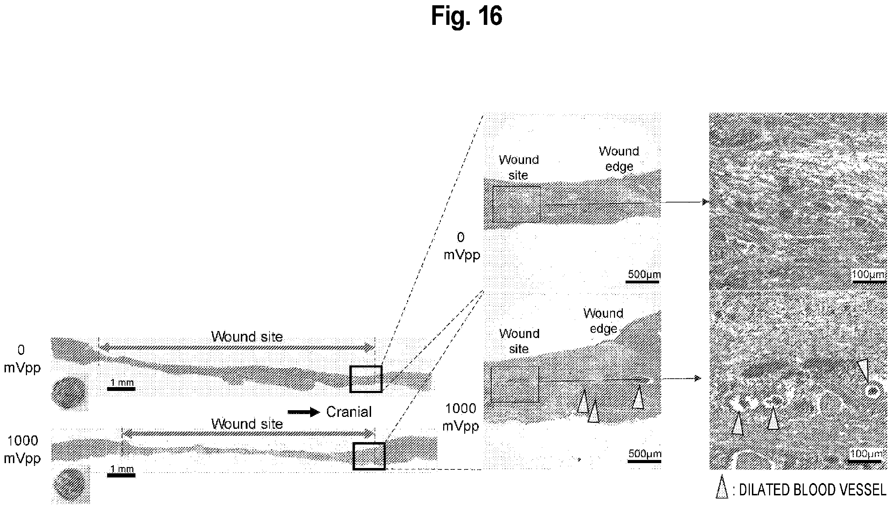

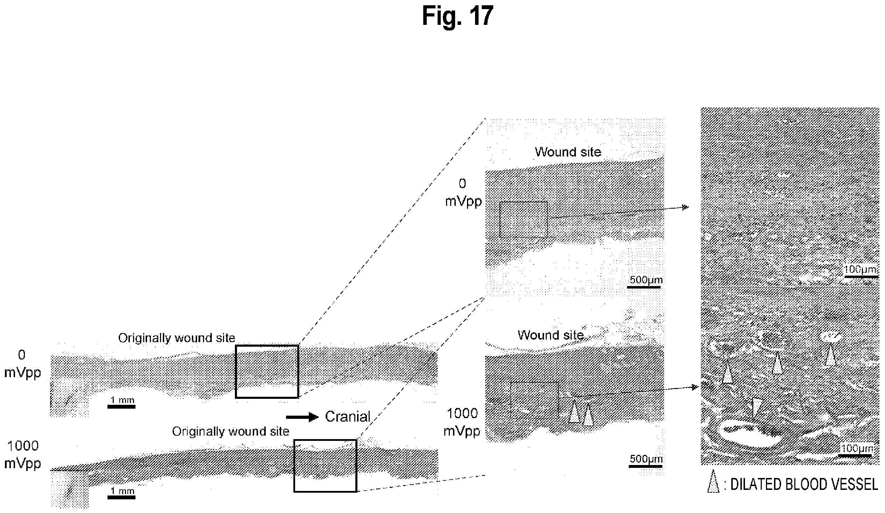

The appearance of the wound site was observed over time, and, as shown in Figure 13, Figure 14, and Figure 15, wound contraction was faster in the diabetic model rats that received vibration than in the diabetic model rats that did not receive vibration. Seven days after wound formation, the tissue of the wound site was observed using hematoxylin-eosin staining as shown in Figure 16: the blood vessels in diabetic model rats that had received a 1000 mVpp vibration were confirmed to be more dilated than in the diabetic model rats that had not received vibration. As shown in Figure 17, the blood vessels in diabetic model rats that had received a 1000 mVpp vibration were also confirmed at 14 days post-wounding to be more dilated than in the diabetic model rats that had not received vibration.

### (Example 4)

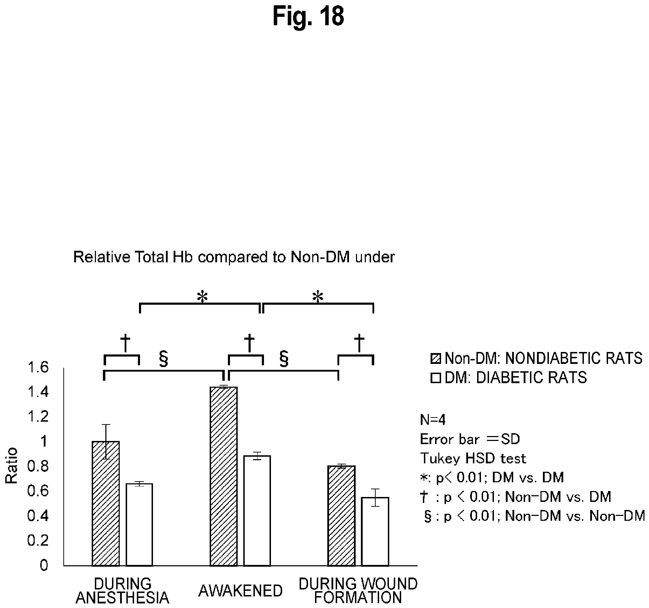

Using nondiabetic rats and diabetic model rats, anesthesia and wound formation were carried out in each as in Example 3, and the tissue total blood volume during anesthesia, the tissue total blood volume during wound formation under anesthesia, and the tissue total blood volume after awakening from anesthesia were measured using a laser tissue blood oxygen monitor (OMEGAMONITOR, BOM-L1 TR SF, OMEGAWAVE, INC.). As shown by the results in Figure 18, in both nondiabetic rats and diabetic model rats, the tissue total blood volume was greater when awakened than during anesthesia. This result indicates that changes in the tissue total blood volume can be more accurately measured when awakened than during anesthesia.

### (Example 5)

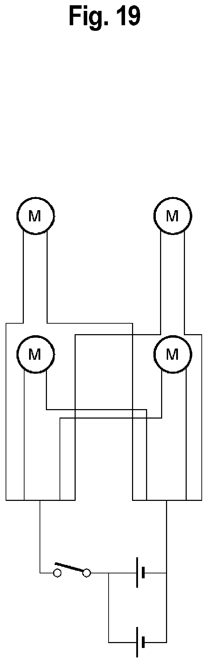

Using C1034 small-scale vibration motors (New Shicoh Motor Co., Ltd.), a vibration device having a circuit equivalent to the circuit diagram shown in Figure 19 was constructed. Proceeding as in Example 3, a full-thickness excisional wound with a diameter of 2 cm was formed on the flank of anesthetized diabetic model rats; a dressing was applied to the wound site of the anesthetized diabetic model rats; and a vibration device was attached on the dressing. The rats were subjected to vibration at 50 Hz and 600 to 1000 mVpp from the vibration device for 40 minutes a day for 7 days. The rats were not anesthetized when the vibration was applied and the rats were in an awake state. In the control group, the vibration device was attached to the rat, but the vibration device was not operated.

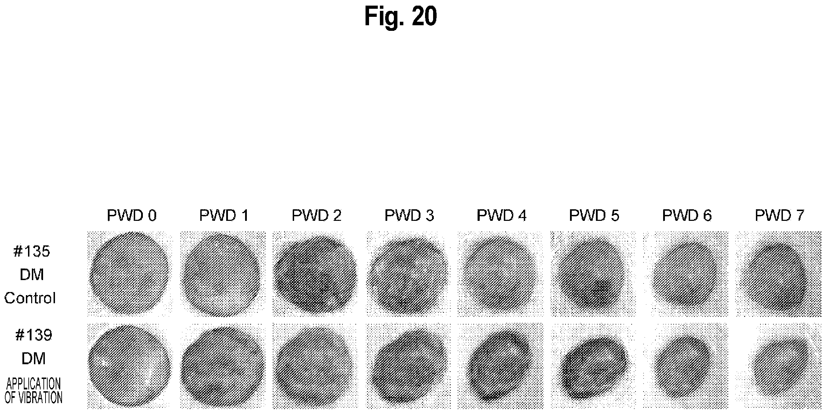

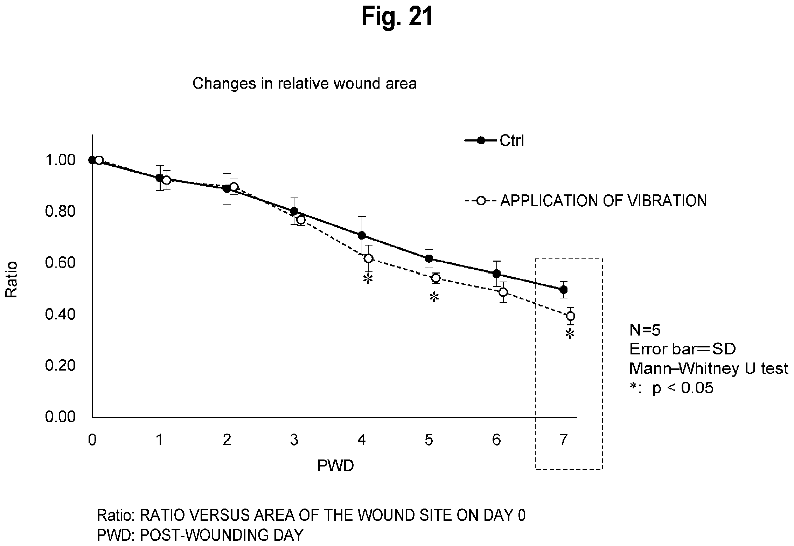

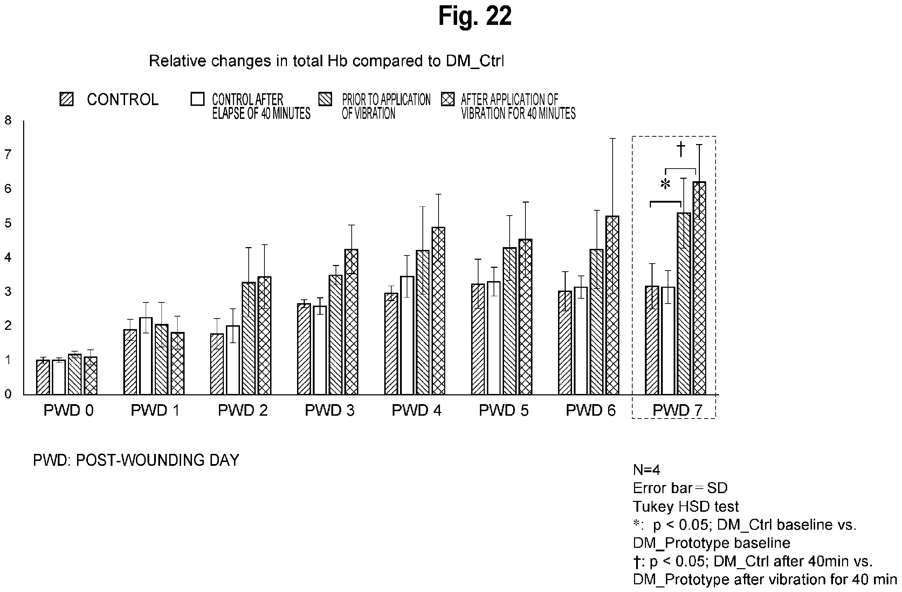

As a result, as shown in Figure 20 and Figure 21, the rats that received vibration exhibited a faster wound site contraction. In addition, Figure 22 provides a graph of the blood volume of the rats using 1 for the blood volume of the control rats on day 0 after wound formation. The blood volume of the rats that received vibration increased each day. It was therefore shown that vibration not only caused a temporary blood vessel dilation, but also increased the steady-state blood volume in the rats.

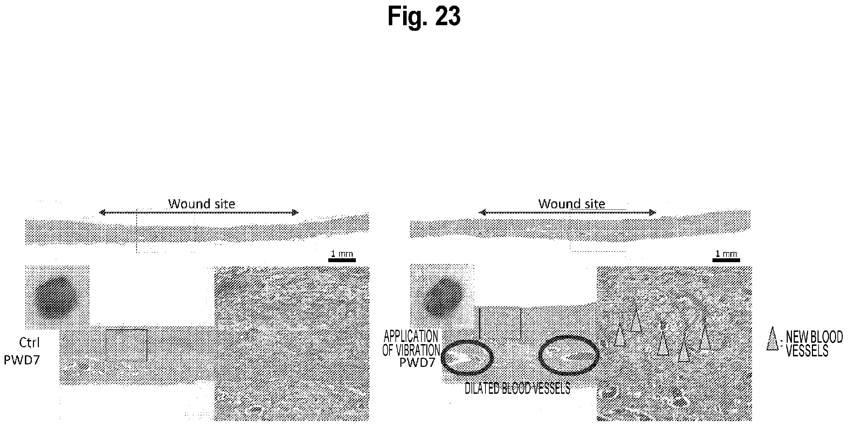

Furthermore, when the wound site tissue was observed by hematoxylin-eosin staining at seven days after wound formation, as shown in Figure 23, greater blood vessel dilation was observed, and numerous new blood vessels were also observed, for the diabetic model rats that received vibration than for the diabetic model rats that had not received vibration. These results indicate that blood vessel dilation and neovascularization led to an increase in the steady-state blood volume of the rats.

### (Example 6)

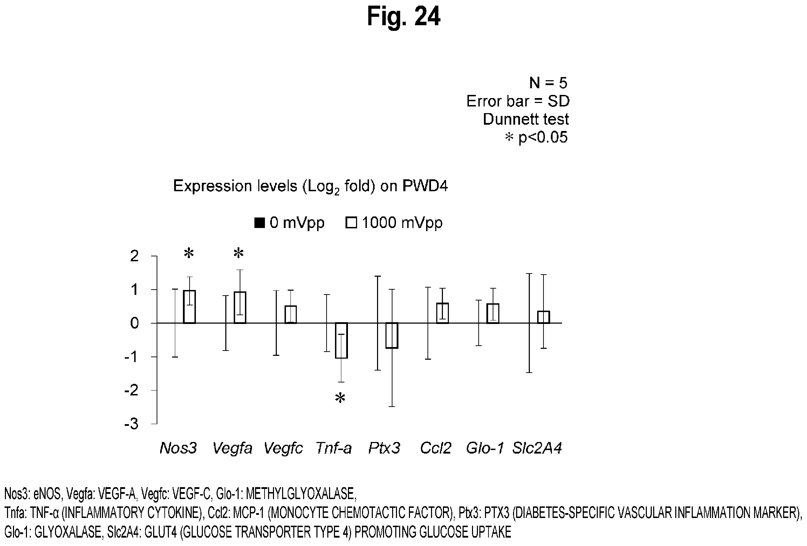

Gene expression was examined on day 4 using the rats subjected to vibration in Example 3, and, as shown in Figure 24, the gene marker NOS3 for the vasodilation-related NO synthetase eNOS and the angiogenesis marker Vegfa were increased by the 1000 mVpp vibration. In addition, the decline in Tnf-a indicates that inflammation was suppressed and the vascular dynamics were improved.

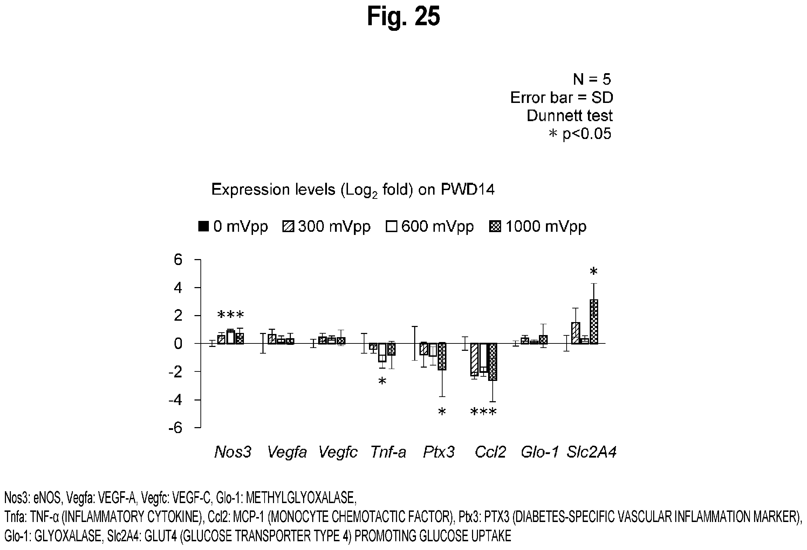

For the rats that received vibration in Example 3, at day 14 wound healing had progressed, and, as shown in Figure 25, inflammatory markers (Tnf-a, Ptx, Ccl2) were significantly reduced in rats that received vibration compared to rats that did not receive vibration. In addition, NOS3, which is related to vasodilation, was significantly increased in rats that received vibration at from 300 mVpp to 1000 mVpp.

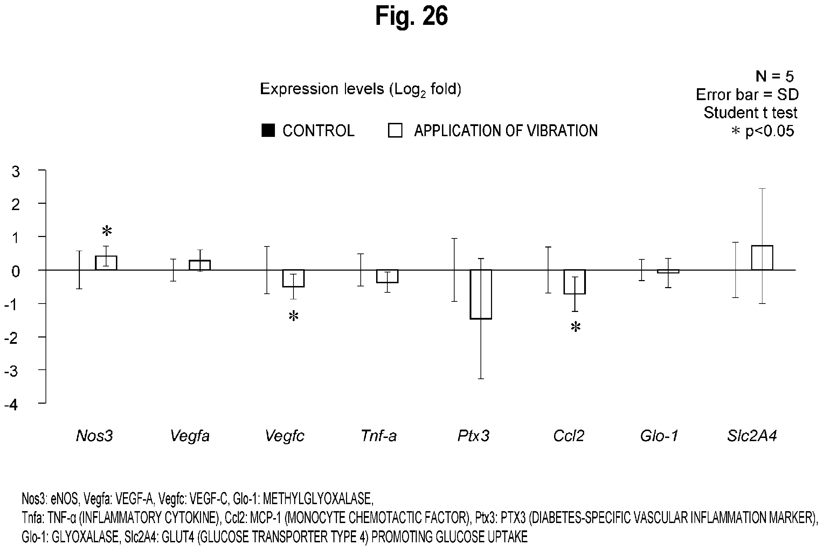

In rats that received vibration in Example 5, gene expression was examined on day 7, and, as shown in Figure 26, it was confirmed that the inflammatory markers were reduced compared to the control, which did not receive vibration.

### (Example 7)

A diabetic model rat was prepared as in Example 3; a full-thickness excisional wound with a diameter of 2 cm was formed on the flank of the diabetic model rat under anesthesia; a dressing (Foamlite, ConvaTec) was applied to the wound site; and the torso was fixed with gauze. The dressing was changed daily. Subsequent to this, a vibrator was placed on the gauze over the wound site each day for 14 days and low-frequency vibration was applied locally to the wound site for 40 minutes while the diabetic model rat was asleep under isoflurane inhalation anesthesia. The frequency of the vibration was 50 Hz, and the vibration intensity was 0 mVpp or 1000 mVpp.

14 days after wound formation, tissue was collected from the wound site and the collected tissue was immersed in a 10% formalin solution overnight at room temperature to fix the tissue. The tissue was then dehydrated using G-Nox (Genostaff), a substitute for ethanol and xylene, and the tissue was subsequently embedded in paraffin to produce a paraffin block of the tissue. The paraffin-embedded tissue was sliced at a thickness of 3 µm to prepare tissue sections.

The tissue sections were immersed in G-Nox 3-times × 5 minutes to remove the paraffin from the tissue sections. The tissue sections were then immersed in ethanol 3-times × 5 minutes to remove the G-Nox from the tissue sections. The tissue sections were subsequently washed with purified water 2-times × 5 minutes. The tissue sections were additionally incubated for 30 minutes in 3% hydrogen peroxide diluted with methanol to inactivate endogenous peroxidase in the tissue sections. The tissue sections were also placed in 0.01 mol/L citrate buffer (pH 6.0) and autoclaved at 121°C for 15 minutes to carry out antigen activation in the tissue sections.

The tissue sections were subsequently washed with phosphate buffered saline (PBS) and reacted overnight with anti-Pentraxin3 (PTX3) antibody (rabbit-polyclonal, 13797-1-AP, Novus Biological) diluted 100-fold with bovine serum albumin/phosphate buffered saline. The anti-PTX3 antibody not bound to antigen was removed from the tissue sections, and the tissue sections were washed with PBS 3-times × 5 minutes.

The tissue sections were additionally incubated with 1000-fold diluted horseradish peroxidase (HRP)-labeled anti-rabbit immunoglobulin antibody (Jackson ImmunoResearch) at room temperature for 1 hour. The HRP was subsequently visualized by reacting the tissue sections with 0.2 mg/mL 3,3'-diaminobenzidine [DAB] (Wako Pure Chemical Industries) in 0.05 mol/L Tris-HCl buffer (pH 7.4) containing 2% hydrogen peroxide, and the reaction was stopped with purified water. Counterstaining with hematoxylin was then performed. The tissue sections were dehydrated with ethanol, made transparent with G-Nox, and then mounted using a mounting agent.

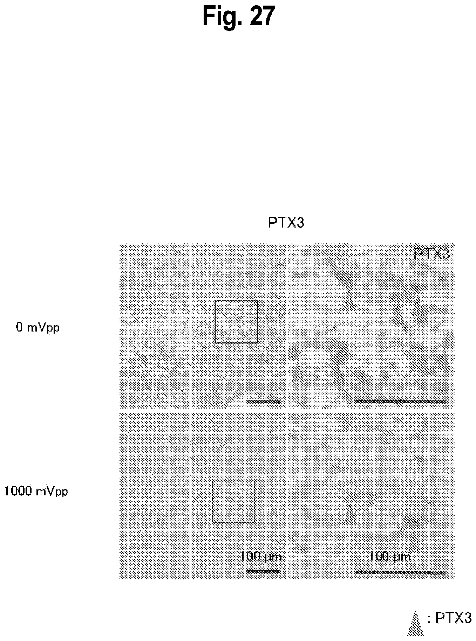

The stained tissue sections were observed under a microscope (BZ-X800, Keyence). As a result, and as shown in Figure 27, it was confirmed that the inflammatory marker PTX3 was significantly reduced in rats that received vibration in comparison to rats that did not receive vibration.

### (Example 8)

Tissue sections were prepared as in Example 7. However, in this example, the endogenous peroxidase in the tissue sections was not inactivated. The tissue sections were washed with phosphate buffered saline (PBS) and reacted overnight with anti-CD68 antibody (CD68/SR-D1 antibody (ED1), mouse monoclonal, NB600-985-0.025, Novus Biological) and anti-CD163 antibody (CD163, EPR19518, rabbit monoclonal, ab182422, Abcam) diluted 100-fold with bovine serum albumin/phosphate buffered saline. CD68 is a marker for M1 macrophages, which promote inflammatory responses. CD163 is a marker for M2 macrophages, which suppress inflammatory responses.

The anti-CD68 antibody and anti-CD163 antibody that were not bound to antigens were subsequently removed from the tissue sections, and the tissue sections were washed with PBS 3 times × 5 minutes. The tissue sections were also incubated at room temperature for 1 hour with 1000-fold diluted green fluorescent dye-labeled anti-rabbit IgG antibody (Alexa Fluor 488, registered trademark, donkey, #711-545-152, Jackson ImmunoResearch) and red fluorescent dye-labeled antimouse IgG antibody (Alexa Fluor 594, registered trademark, donkey, #715-585-151, Jackson ImmunoResearch). The tissue sections were subsequently washed with PBS; the nuclei were stained with blue fluorescent dye using DAPI; and the tissue sections were mounted.

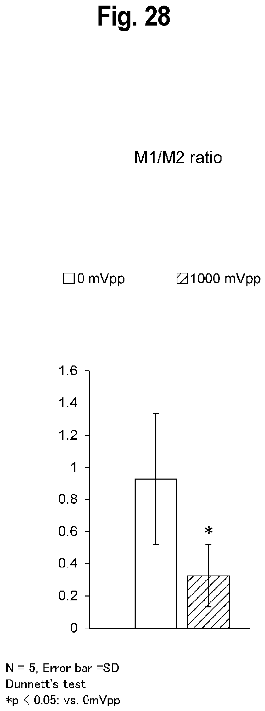

The stained tissue sections were observed under a microscope (BZ-X800, Keyence). Cells emitting green fluorescence were CD163 positive cells. Cells emitting red fluorescence were CD68 positive cells. The number of CD68 positive cells and the number of CD163 positive cells were counted. The value obtained by dividing the number of CD68 positive cells by the number of CD163 positive cells (M1/M2 macrophage ratio) was calculated. A smaller M1/M2 ratio indicates a greater improvement in inflammation. As a result, and as shown in Figure 28, M1/M2 was significantly reduced in rats that received vibration in comparison to rats that did not receive vibration.

### Reference Signs List

- 11: Glucose concentration sensor
- 12: Blood flow sensor
- 21: Vibrator
- 31: Control device
- 41: Dressing

## Claims

1. A body surface ulcer healing promotion device comprising:
a glucose concentration sensor that detects a glucose concentration in an exudate of an ulcer on a body surface;
a vibrator for applying vibration to the ulcer; and
a control device that controls the vibration of the vibrator on a basis of the glucose concentration in the exudate.

2. The body surface ulcer healing promotion device according to claim 1, wherein the ulcer is an ulcer caused by diabetes.

3. The body surface ulcer healing promotion device according to claim 1, wherein the ulcer is a foot ulcer caused by diabetes.

4. The body surface ulcer healing promotion device according to claim 1, wherein the vibrator is provided in plurality.

5. The body surface ulcer healing promotion device according to claim 1, wherein the control device controls the vibration of the vibrator such that cellular arginine production is promoted.

6. The body surface ulcer healing promotion device according to claim 1, wherein the control device controls the vibration of the vibrator such that the glucose concentration becomes equal to or greater than a concentration at which blood flow is increased.

7. The body surface ulcer healing promotion device according to claim 1, wherein the control device causes the vibrator to vibrate until the glucose concentration becomes equal to or less than a concentration at which the amount of cellular arginine production becomes equal to or greater than a threshold value.

8. The body surface ulcer healing promotion device according to claim 1, further comprising a blood flow sensor that detects blood flow in the vicinity of the ulcer, wherein the control device controls the vibration of the vibrator on a basis of the blood flow.

9. The body surface ulcer healing promotion device according to claim 8, wherein the control device controls the vibration of the vibrator so as to increase the blood flow.
